# EUROPEAN PATENT SPECIFICATION

(11) **EP 4 633 576 B1**
(45) Date of publication and mention of the grant of the patent: **11.11.2026**
(21) Application number: 23828753.6
(22) Date of filing: 14.12.2023
(51) Int. Cl.: A61K 8/02, A61K 8/06, A61K 8/24, A61K 8/34, A61K 8/44, A61K 8/46, A61K 8/92, A61Q 1/00, A61Q 1/02, A61Q 5/00, A61Q 19/00, A61Q 19/08

(54) **COMPOSITION COMPRISING AT LEAST ONE SATURATED FATTY ALCOHOL, AT LEAST ONE ANIONIC SURFACTANT, AT LEAST ONE POLYOL AND AT LEAST ONE NON-SILICONE OIL, THE COMPOSITION BEING FREE OF SILICONE COMPOUNDS**
ZUSAMMENSETZUNG, DIE MINDESTENS EINEN GESÄTTIGTEN FETTALKOHOL, MINDESTENS EIN ANIONISCHES TENSID, MINDESTENS EIN POLYOL UND MINDESTENS EIN SILIKONFREIES ÖL ENTHÄLT, WOBEI DIE ZUSAMMENSETZUNG FREI VON SILIKONVERBINDUNGEN IST
COMPOSITION COMPRENANT AU MOINS UN ALCOOL GRAS SATURÉ, AU MOINS UN TENSIOACTIF ANIONIQUE, AU MOINS UN POLYOL ET AU MOINS UNE HUILE NON SILICONÉE, LA COMPOSITION ÉTANT EXEMPTE DE COMPOSÉS SILICONÉS

(30) Priority: 15.12.2022 FR 2213440
(43) Date of publication of application: 22.10.2025
(73) Proprietor: L'OREAL, 75008 Paris (FR)
(72) Inventor: FALIP, Anne, 94152 Chevilly Larue (FR); LIBII, Majolie, 94152 Chevilly Larue (FR); GUIRAMAND, Carole, 94152 Chevilly Larue (FR)
(74) Representative: Ipsilon
(86) International application number: PCT/EP2023/085801
(87) International publication number: WO 2024/126678

(56) References cited:
- WO-A1-2013/102566
- WO-A1-2020/002538
- US-A1- 2016 120 788
- US-A1- 2017 326 060
- US-A1- 2020 109 231

## Description

### Technical field

The present invention relates to a composition, preferably a cosmetic composition, in the form of an oil-in-water emulsion, in which an oily phase is dispersed in an aqueous phase, comprising:
(a) at least one saturated C16-C22 and preferably C16-C18 fatty alcohol;
(b) at least one anionic surfactant including at least one C16-C22 and preferably C16-C18 hydrocarbon-based chain;
(c) at least one polyol present in an amount strictly greater than 15% by weight relative to the total weight of the composition, and
(d) at least one non-silicone oil present in an amount of greater than or equal to 8% by weight relative to the total weight of the composition, the composition being free of silicone compounds.

### Prior art

The particular type of galenic form targeted by the present invention, namely an oil-in-water emulsion notably requiring the presence of at least one saturated C16-C22 fatty alcohol and at least one anionic surfactant including at least one C16-C22 hydrocarbon-based chain, allows active agents to be conveyed while at the same time affording advantageous sensory properties that are appreciated by users when it is applied to keratin materials.

Oil-in-water emulsion compositions notably requiring the presence of at least one saturated C16-C22 fatty alcohol and at least one anionic surfactant including at least one C16-C22 hydrocarbon-based chain are described in particular in WO 2018/108878 and WO 2020/002538.

However, these compositions address very specific objectives. Their purpose is notably, respectively, to mask skin imperfections, moisturize the skin and/or treat the signs of skin ageing, in particular the appearance of fine lines and deep wrinkles, particularly as they increase with age.

The term "keratin material" notably means the skin, the scalp, keratin fibres such as the eyelashes, the eyebrows, head hair, bodily hair, the nails, and mucous membranes such as the lips, and more particularly the skin (body, face, neck, area around the eyes, eyelids). However, it is sometimes difficult to reconcile the requirements in terms of sensory properties, texture and stability. In particular, it is desirable to have available compositions that are capable of maintaining the same viscosity over time, in particular a viscosity which neither increases nor decreases over time.

### Disclosure of the invention

The aim of the present invention is to solve the abovementioned technical problems.

In the light of the foregoing, it is thus clear that there is still a need for compositions with advantageous sensory properties, and in particular good glidance and a non-tacky aspect, and a reduction or absence of soaping, while at the same time retaining satisfactory stability, in particular stability evaluated at 1 month and/or 2 months of storage, in particular macroscopically and microscopically after two months of storage under temperature conditions ranging from 4°C to 45°C, in particular at 4°C, room temperature (generally between 20°C and 25°C), and 45°C.

Finally, consumers are nowadays very sensitive with regard to the environmental impact of the products used.

Consequently, it remains very difficult to obtain cosmetic compositions which offer particular sensory properties in terms of a glidant and non-tacky feel on application, with little soaping, while at the same time retaining good stability over time.

### Summary of the invention

Thus, according to a first aspect, the present invention relates to a composition, preferably a cosmetic composition, in the form of an oil-in-water emulsion, in which an oily phase is dispersed in an aqueous phase, comprising:
(a) at least one saturated C16-C22 and preferably C16-C18 fatty alcohol;
(b) at least one anionic surfactant including at least one C16-C22 and preferably C16-C18 hydrocarbon-based chain;
(c) at least one polyol present in an amount strictly greater than 15% by weight relative to the total weight of the composition, and
(d) at least one non-silicone oil present in an amount of greater than or equal to 8% by weight relative to the total weight of the composition,
the composition being free of silicone compounds.

For the purposes of the present invention, the term "free of silicone compounds" refers to a composition according to the invention which comprises less than 2% by weight, preferably less than 1% by weight, relative to the total weight of the composition, and more preferentially is free (devoid) of silicone compounds.

The term "silicone compounds" (also called silicones) means any silicone compounds that are liable to be used in the cosmetic and/or dermatological fields, i.e. inorganic compounds consisting at least of a mixture of silicon and oxygen, notably including silicone oils, silicone surfactants, dimethylsiloxane polymers, volatile or functionalized silicones, silicone elastomers, silicone resins, phenyl silicones or amino silicones.

As illustrated in the examples hereinbelow, the Applicant has discovered, surprisingly, that it is possible to obtain compositions with advantageous sensory properties, and in particular good glidance and a non-tacky aspect, with little soaping, while at the same time retaining satisfactory stability over time.

The compositions according to the present invention advantageously enable ingredients of various kinds such as cosmetic active agents, sunscreens, particles, polymers, dyes, nacres or pigments and fragrances to be conveyed, depending on the various benefit(s) desired. The compositions according to the present invention also have the advantage of affording an anti-ageing effect.

According to a particular embodiment, the present invention concerns a composition, notably a cosmetic composition, in the form of an oil-in-water emulsion, in which an oily phase is dispersed in an aqueous phase, comprising:
(a) at least one saturated C16-C22 and preferably C16-C18 fatty alcohol;
(b) at least one anionic surfactant including at least one C16-C22 and preferably C16-C18 hydrocarbon-based chain;
(c) at least one polyol present in an amount strictly greater than 15% by weight relative to the total weight of the composition, and
(d) at least one non-silicone oil present in an amount of greater than or equal to 8% by weight relative to the total weight of the composition,
the composition being free of silicone compounds,

in which the anionic surfactant(s) including at least one C16-C22 hydrocarbon-based chain are chosen from the surfactants of general formula (I): RCOY(CH₂)ₙSO₃M in which R represents a saturated, linear or branched C16-C22 alkyl group; Y represents -O- or -NR₁-with R₁ representing a linear or branched C1-C3 alkyl group; M is chosen from the group formed by hydrogen, alkali metals, alkaline-earth metals, the ammonium group and organic amines; n is an integer ranging from 1 to 3.

The invention also relates to a process for the cosmetic treatment of a keratin material in which a composition as defined previously is applied to the keratin material.

Other characteristics, aspects and advantages of the invention will become apparent on reading the detailed description that follows.

### Detailed description

The composition according to the invention is cosmetic and/or dermatological, and preferably is cosmetic.

The composition according to the invention is generally suitable for topical application to the skin and thus generally comprises a physiologically acceptable medium, i.e. a medium that is compatible with the skin.

The term *"cosmetic"* refers to a composition that is compatible with the skin, mucous membranes and the integuments. The composition according to the invention is non-therapeutic.

It is preferably a cosmetically acceptable medium, i.e. a medium which has a pleasant colour, odour and feel and which does not cause any unacceptable discomfort, i.e. stinging, tautness or redness, liable to discourage the user from applying this composition.

In the text hereinbelow, the expression "at least one" is equivalent to "one or more" and, unless otherwise indicated, the limits of a range of values are included in that range.

### Saturated C16-C22 fatty alcohol

The term "saturated fatty alcohol" means any alcohol comprising a linear saturated (containing no covalent double or triple bonds) hydrocarbon-based chain, in particular a linear alkyl radical, said chain comprising between 16 and 22 carbon atoms and a hydroxyl function.

The term "hydrocarbon-based chain" means an organic group predominantly consisting of hydrogen atoms and carbon atoms.

The saturated fatty alcohol(s) present in the composition in accordance with the invention comprise between 16 and 22 carbon atoms, preferably between 16 and 18 carbon atoms. The saturated C16-C22 fatty alcohol(s) used in the compositions in accordance with the present invention are solid at room temperature and advantageously bear a chain-end -OH group.

The saturated fatty alcohol(s) that are of use in the context of the present invention may notably be chosen from cetyl alcohol or hexadecanol (C₁₆), stearyl alcohol or octadecanol (C₁₈), or behenyl alcohol (C₂₂) which are solid at room temperature and advantageously bear a chain-end -OH group.

Particularly preferably, use will be made of one or more alcohols chosen from cetyl alcohol, stearyl alcohol, and mixtures thereof such as cetylstearyl alcohol.

Preferably, the composition according to the invention comprises a mixture of C₁₆ and C₁₈ fatty alcohols; in particular, the composition comprises cetylstearyl alcohol.

More preferably, the mixture of C₁₆ and C₁₈ fatty alcohols is used in a C₁₆/C₁₈ mass ratio ranging from 20/80 to 80/20 and advantageously in a C₁₆/C₁₈ mass ratio equal to 50/50.

The amount of fatty alcohols in the composition in accordance with the invention is between 2.5% and 10% by weight, preferably between 2.5% and 8% by weight and even more preferentially between 2.7% and 7% by weight, relative to the total weight of the composition.

### Anionic surfactants including at least one C16-C22 hydrocarbon-based chain

The anionic surfactant(s) that are useful in the context of the invention comprise at least one saturated (not containing any covalent double or triple bonds) or unsaturated (which may contain one or more covalent double and/or triple bonds), preferably saturated, linear hydrocarbon-based chain, in particular a linear alkyl radical, said chain comprising from 16 to 22 carbon atoms, preferably from 16 to 18 carbon atoms. Preferably, the anionic surfactant(s) that are useful in the context of the invention comprise only one saturated or unsaturated linear hydrocarbon-based chain.

According to a particular embodiment, the anionic surfactant(s) that are useful in the context of the invention contain only one C16-C22 hydrocarbon-based chain.

The term "hydrocarbon-based chain" means an organic group predominantly consisting of hydrogen atoms and carbon atoms.

According to a particular embodiment of the invention, the anionic surfactant(s) including at least one C16-C22 hydrocarbon-based chain are chosen from surfactants comprising at least one sulfonate function, acylglutamates, and mixtures thereof.

According to a preferred embodiment of the invention, the anionic surfactant(s) comprising at least one sulfonate function are chosen from (C16-C22)alkylsulfonates, (C16-C22)alkylamidesulfonates, (C16-C22)alkylarylsulfonates, (C16-C22)alkylsulfoacetates, N-acyl(C16-C22)-N-(C1-C6)alkyltaurates, (C16-C22)acylisethionates, (C16-C22)alkylsulfolaurates, and mixtures thereof.

Preferentially, the anionic surfactant(s) comprising at least one sulfonate function are chosen from:
- acylisethionates, the linear or branched acyl group comprising from 16 to 22 carbon atoms, preferably from 16 to 18 carbon atoms;
- N-acyl-N-alkyltaurates, the linear or branched acyl group comprising from 16 to 22 carbon atoms, preferably from 16 to 18 carbon atoms, and the linear or branched alkyl group comprising from 1 to 6 carbon atoms, or cyclic group comprising from 3 to 6 carbon atoms; preferably, the alkyl group is a methyl,
- and mixtures thereof;
in particular in the form of alkali metal or alkaline-earth metal, ammonium or amino alcohol salts.

As N-acyl-N-alkyltaurates, mention may be made of sodium palmitoyl methyltaurate sold under the name Nikkol PMT^{®} by the company Nikkol; the sodium salt of N-stearoyl N-methyl taurate sold under the name Nikkol SMT by the company Nikko.

The acyl glutamate(s) may be chosen from acyl glutamates in which the acyl group comprises from 16 to 18 carbon atoms.

Examples of acyl glutamates that may notably be mentioned include palmitoyl glutamic acid, stearoyl glutamic acid, behenoyl glutamic acid, olivoyl glutamic acid, and the salts of these acids, notably the alkali metal salts such as the Na, Li or K and preferably Na or K salts, the alkaline-earth metal salts such as the Mg salts or the ammonium salts of said acids.

Mention may be made, for example, of the compounds having the INCI name sodium stearoyl glutamate, sodium olivoyl glutamate, and mixtures thereof.

As acylglutamic acid salts, mention may also be made of sodium stearoyl glutamate, such as the product sold under the reference Acylglutamate HS 11 by the company Ajinomoto and disodium hydrogenated stearoyl glutamate, such as the product sold under the reference Acylglutamate HS-21 by the company Ajinomoto.

According to a particular embodiment of the invention, the anionic surfactant(s) including at least one C16-C22 hydrocarbon-based chain are chosen from the surfactants of general formula (I): RCOY(CH₂)ₙSO₃M in which R represents a saturated, linear or branched C16-C22 alkyl group; Y represents -O- or -NR₁- with R₁ representing a linear or branched C1-C3 alkyl group; M is chosen from the group formed by hydrogen, alkali metals, alkaline-earth metals, the ammonium group and organic amines; n is an integer ranging from 1 to 3. Preferably, the anionic surfactant of general formula (I) is N-stearoyl-N-methyltaurate, with M corresponding to Na.

The composition according to the invention advantageously comprises from 0.05% to 5%, preferably from 0.1% to 2% and even more preferentially from 0.1% to 1.5% by weight, relative to the total weight of the composition, of one or more anionic surfactants including at least one C16-C22 hydrocarbon-based chain as defined previously.

In the compositions in accordance with the invention, the fatty alcohols (a)/anionic surfactants (b) mass ratio may be between 10:1 and 6:4, preferably between 19:2 and 7:3, and even more preferentially between 9:1 and 4:1.

Without wishing to be bound by any theory, it appears that the fatty alcohol(s) and the anionic surfactant(s) including at least one C16-C22 hydrocarbon-based chain form, with the hydrophilic phase, an alpha crystalline phase, also referred to as alpha gel. This phase may notably be characterized by DSC analysis and by X-ray diffraction at a temperature lower than the melting point of the alpha gel phase.

Thus, the composition in accordance with the invention is capable of forming crystalline lamellar phases.

According to a particular embodiment of the invention, the compositions of the invention contain at least one swollen alpha-crystalline phase having a swelling period of greater than 10 nm, preferably greater than 12 nm and even more preferentially greater than or equal to 15 nm. The period is defined as being the sum of the thickness of a bilayer formed by the fatty alcohols (a) and the anionic surfactants (b) and the thickness of the water layer between two leaflets, this unit being repeated several times.

According to a particular embodiment, in a composition according to the invention, the difference between the number of carbon atoms in the hydrocarbon-based chain of the anionic surfactant(s) (b) and the number of carbon atoms in the fatty alcohol(s) (a) must not differ by more than 5 carbon atoms, and is preferentially less than or equal to 4.

### Polyols

The composition according to the invention comprises at least one polyol in a content strictly greater than 15% by weight relative to the total weight of the composition.

Polyols are defined as being organic molecules comprising at least two hydroxyl (OH) functions.

For the purposes of the invention, the term "polyol" means:
- a saturated or unsaturated, linear or branched hydrocarbon-based chain comprising at least two hydroxyl functions; or
- a saturated, linear or branched hydrocarbon-based chain in which one or more carbon atoms are replaced with an oxygen atom and which comprises at least two hydroxyl functions, for instance polyethylene glycols (PEGs) containing from 4 to 8 ethylene glycol units. Preferably, the polyol(s) bear a saturated, linear or branched hydrocarbon-based chain. Advantageously, the polyol(s) comprise a number of carbon atoms ranging from 2 to 20, preferably from 2 to 10, and comprise from 2 to 12 and better still from 2 to 8 hydroxyl functions.

The polyol(s) may be chosen from ethylene glycol, propylene glycol, 1,3-propanediol, isoprene glycol, butylene glycol, dipropylene glycol, polypropylene glycol, caprylyl glycol, glycerol, diglycerol, erythritol, pentaerythritol, arabitol, adonitol, sorbitol, dulcitol, maltitol and panthenol.

Among these polyols, glycerol, caprylyl glycol, propylene glycol, dipropylene glycol, butylene glycol, propane-1,3-diol, and mixtures thereof, are preferentially chosen.

According to one embodiment, the polyol(s) comprise from 2 to 12 hydroxyl functions, preferably from 2 to 8 hydroxyl functions, and in particular are chosen from ethylene glycol, propylene glycol, propane-1,3-diol, isoprene glycol, butylene glycol, dipropylene glycol, polypropylene glycol, caprylyl glycol, glycerol, diglycerol, erythritol, pentaerythritol, arabitol, adonitol, sorbitol, dulcitol, maltitol, panthenol and mixtures thereof, preferably glycerol, caprylyl glycol, propylene glycol, dipropylene glycol, butylene glycol, propane-1,3-diol and mixtures thereof, and even more preferentially glycerol, caprylyl glycol and mixtures thereof.

Preferably, the polyol is chosen from glycerol, caprylyl glycol and mixtures thereof.

According to another particular embodiment of the invention, the polyol(s) may be present in the composition in an amount of between 15.1% and 50% by weight, preferably between 18% and 45% by weight, even more preferentially between 19% and 40% by weight and better still between 20% and 35% by weight relative to the total weight of the composition.

### Non-silicone oils

The composition according to the invention comprises at least one non-silicone oil in a content of greater than or equal to 8% by weight relative to the total weight of the composition.

The term *"oil"* means a water-immiscible non-aqueous compound that is liquid at room temperature (20°C) and at atmospheric pressure (760 mmHg).

The composition according to the invention may in particular comprise hydrocarbon-based oils.

They may be of animal, plant, mineral or synthetic origin.

For the purposes of the present invention, the term *"hydrocarbon-based oil"* means an oil mainly containing hydrogen and carbon atoms.

The term *"non-silicone oil"* means an oil not comprising any silicon atoms and notably no Si-O groups.

The oils may optionally comprise oxygen, nitrogen, sulfur and/or phosphorus atoms, for example in the form of hydroxyl or acid radicals.

Hydrocarbon-based oils that may notably be mentioned include:
- branched alkanes comprising more than 8 carbon atoms, notably C₈-C₁₆ alkanes, for instance C₈-C₁₆ isoalkanes (also known as isoparaffins), isododecane, isodecane, isohexadecane and, for example, the oils sold under the trade names Isopar or Permethyl, branched C₈-C₁₆ esters, for instance isohexyl neopentanoate, and mixtures thereof,
- linear alkanes comprising more than 8 carbon atoms, in particular from 10 to 30 carbon atoms, in particular from 10 to 26 carbon atoms, and more particularly from 10 to 20 carbon atoms, for instance n-dodecane (C₁₂) and n-tetradecane (C₁₄) sold by Sasol under the respective references Parafol 12-97 and Parafol 14-97, and also mixtures thereof, the undecane-tridecane mixture, mixtures of n-undecane (C₁₁) and of n-tridecane (C₁₃) obtained in examples 1 and 2 of patent application WO 2008/155059 from the company Cognis, and mixtures thereof; or a mixture of alkanes of 15 to 19 carbon atoms (called C15-C19 alkane), for example the products sold under the references Emogreen L19 and Emosmart L19 from SEPPIC,
- hydrocarbon-based oils of animal origin,
- linear or branched hydrocarbons of mineral or synthetic origin, such as petroleum jelly, polydecenes, hydrogenated polyisobutene such as Parleam, and squalane, and mixtures thereof,
- hydrocarbon-based oils of plant origin, such as glyceride triesters, which are generally triesters of fatty acids and of glycerol, the fatty acids of which may have varied chain lengths of from 4 to 24 carbon atoms, these chains possibly being linear or branched, and saturated or unsaturated; these oils are notably wheatgerm oil, rice bran oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, alfalfa oil, poppyseed oil, pumpkin oil, marrow oil, blackcurrant oil, evening primrose oil, millet oil, barley oil, quinoa oil, rye oil, safflower oil, candlenut oil, passionflower oil, musk rose oil, *Limnanthes alba* seed oil (INCI name: *Limnanthes alba* (Meadowfoam) Seed Oil); or caprylic/capric acid triglycerides such as those sold by the company Stéarinerie Dubois or those sold under the names Miglyol 810, 812 and 818 by the company Dynamit Nobel. Mention may also be made of cocoyl caprylate/caprate, for example sold under the name Cetiol LC by the company Cognis or under the name DUB 810 C by the company Stéarinerie Dubois,
- synthetic ethers containing from 10 to 40 carbon atoms, such as dicapryl ether,
- synthetic esters, such as the oils of formula R₁COOR₂, in which R₁ represents a linear or branched fatty acid residue including from 1 to 40 carbon atoms and R₂ represents a hydrocarbon-based chain that is notably branched, containing from 1 to 40 carbon atoms, with the proviso that R₁ + R₂ is greater than or equal to 10, for instance purcellin oil (cetostearyl octanoate), isopropyl myristate, myristyl myristate, isopropyl palmitate, alkyl benzoates containing between 12 and 15 carbon atoms, such as the product sold under the trade name Finsolv TN or Witconol TN by the company Witco or Tegosoft TN by the company Evonik Goldschmidt, 2-ethylphenyl benzoate, such as the commercial product sold under the name X-Tend 226 by the company ISP, isopropyl lanolate, hexyl laurate, diisopropyl adipate, isononyl isononanoate, oleyl erucate or (Z)-octadec-9-enyl (Z)-docos-13-enoate, 2-ethylhexyl palmitate, isostearyl isostearate, diisopropyl sebacate such as the product sold under the name Dub Dis by the company Stéarinerie Dubois, alcohol or polyalcohol octanoates, decanoates or ricinoleates, such as propylene glycol dioctanoate; hydroxylated esters, such as isostearyl lactate, diisostearyl malate; and pentaerythritol esters, in particular pentaerythrityl tetraoctanoate (INCI name: Pentaerythrityl Tetraethylhexanoate); dipentaerythrityl hexacaprylate/hexacaprate, citrates, such as the ester of C₃-C₂₂ tricarboxylic acid and C₁-C₆ alcohols having the INCI name Triethyl Citrate, for example the product sold under the name Citrofol AI Extra by the company Jungbunzlauer; tartrates such as linear dialkyl tartrates containing 12 or 13 carbon atoms, for example those sold under the name Cosmacol ETI by the company Enichem Augusta Industriale, and also linear dialkyl tartrates containing between 14 and 15 carbon atoms, for example those sold under the name Cosmacol ETL by the same company, and acetates,
- polyol esters and pentaerythritol esters, for instance dipentaerythrityl tetrahydroxystearate/tetraisostearate,
- esters of diol dimer and diacid dimer, where appropriate esterified on their free alcohol or acid function(s) with acid or alcohol radicals, in particular dimer dilinoleate esters; more particularly chosen from the esters having the following INCI nomenclature: bis-behenyl/isostearyl/phytosteryl dimer dilinoleyl dimer dilinoleate (Plandool G), phytosteryl/isostearyl/cetyl/stearyl/behenyl dimer dilinoleate (Plandool H or Plandool S), and mixtures thereof,
- fatty amides, such as isopropyl N-lauroyl sarcosinate, for example the product sold under the trade name Eldew SL205 from Ajinomoto,
- fatty alcohols that are liquid at room temperature, with a branched and optionally unsaturated carbon-based chain containing from 12 to 26 carbon atoms, for instance octyldodecanol, 2-butyloctanol or 2-undecylpentadecanol,
- higher C₁₉-C₂₂ fatty acids;
- carbonates, such as dicaprylyl carbonate, for example the product sold under the name Cetiol CC by the company Cognis.

According to a particular embodiment of the invention, the non-silicone oil(s) are chosen from branched or linear alkanes comprising more than 8 carbon atoms, hydrocarbon-based oils of animal origin, linear or branched hydrocarbons of mineral or synthetic origin, hydrocarbon-based oils of plant origin, synthetic ethers including from 10 to 40 carbon atoms, and synthetic esters of formula R₁COOR₂, in which R₁ represents a residue of a linear or branched fatty acid containing from 1 to 40 carbon atoms and R₂ represents a hydrocarbon-based chain, notably a branched chain containing from 1 to 40 carbon atoms, on condition that R₁ + R₂ is greater than or equal to 10, polyol esters and pentaerythritol esters, diol dimer and diacid dimer esters, fatty amides, fatty alcohols that are liquid at room temperature, with a branched and optionally unsaturated carbon-based chain containing from 12 to 26 carbon atoms, higher C₁₉-C₂₂ fatty acids, carbonates and mixtures thereof; in particular from oleyl erucate, branched or linear alkanes including more than 8 carbon atoms, and mixtures thereof; more particularly from oleyl erucate, a mixture of alkanes of 15 to 19 carbon atoms, and mixtures thereof.

The composition according to the invention advantageously comprises from 8% to 40% by weight, preferably from 9% to 25% by weight, and even more preferentially from 10% to 15% by weight of non-silicone oil(s) relative to the total weight of the composition.

### Waxes

A composition according to the invention may comprise at least one wax, notably a wax of plant origin with a melting point above 25°C.

For the purposes of the present invention, the term *"wax"* means a lipophilic compound that is solid at room temperature (25°C), with a solid/liquid reversible change of state.

For the purposes of the invention, the melting point corresponds to the temperature of the most endothermic peak observed on thermal analysis (differential scanning calorimetry or DSC) as described in the standard ISO 11357-3:1999.

The melting point of the wax may be measured using a differential scanning calorimeter (DSC), for example the calorimeter sold under the name MDSC 2920 by the company TA instruments.

The measuring protocol is as follows: a sample of 5 mg of wax placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

As waxes of plant origin with a melting point above 25°C that may be used according to the invention, mention may be made of:
- carnauba wax,
- candelilla wax, in particular the commercial reference Candelilla Wax SP 75 G from the company Strahl & Pitsch,
- lanolins and lanolin derivatives such as acetylated lanolins, oxypropylenated lanolins or isopropyl lanolate, and mixtures thereof,
- rice bran wax,
- ouricury wax,
- esparto grass wax,
- cork fibre wax,
- sugar cane wax,
- Japan wax,
- sumac wax,
- montan wax,
- orange wax, in particular the product sold under the reference Orange Peel Wax by the company Koster Keunen,
- laurel wax,
- sunflower wax,
- lemon wax,
- olive wax,
- berry wax,
- beeswax, in particular the product sold under the name White Beeswax SP 453P by the company Strahl & Pitsch or Cerabeil Lor by the company Baerlocher,
- buckwheat wax, in particular the product sold by the company Codif,
- waxes obtained by hydrogenation of olive oil esterified with C₁₂ to C₁₈ fatty alcohols, in particular those sold by the company Sophim in the Phytowax range (12L44, 14L48, 16L55 and 18L57),
- partially hydrogenated wax esters of jojoba oil,
- hydrogenated jojoba oil,
- isomerized jojoba oil, in particular the trans-isomerized partially hydrogenated jojoba oil manufactured or sold by the company Desert Whale under the trade name Iso-Jojoba-500,
- fatty acid esters of polyglycerol, in particular the mixture of three waxes jojoba esters & *Helianthus annuus* seed wax *& Acacia decurrens* extract and polyglyceryl, in particular the product sold under the name Hydracire S or Acticire^{®} by the company Gattefossé,
- hydrogenated glycerol esters, in particular the product sold under the name Cegesoft HF 52 by Cognis (BASF), a mixture of hydrogenated rapeseed and palm oils, or the product sold under the name Softisan 100 Cremer by the company Oleo,
- partially hydrogenated olive oil, in particular the compound sold under the name Beurrolive by the company Soliance,
- hydrogenated sunflower oil,
- hydrogenated castor oil,
- hydrogenated castor oil derivatives, in particular Thixinr from Rheox,
- hydrogenated coconut kernel oil,
- hydrogenated palm oil,
- hydrogenated coconut oil,
- hydrogenated cocoglycerides,
- the waxes obtained by hydrogenation of castor oil esterified with cetyl alcohol,
- behenyl alcohol,
- and/or mixtures thereof.

According to a preferred embodiment, a composition according to the invention comprises at least one wax of plant origin with a melting point above 25°C chosen from carnauba wax, partially hydrogenated wax esters of jojoba oil, hydrogenated jojoba oil (INCI name: Hydrogenated jojoba oil), fatty acid esters of polyglycerol, hydrogenated cocoglycerides, behenyl alcohol, and mixtures thereof.

More preferentially, a composition according to the invention comprises at least hydrogenated jojoba oil.

According to a particular embodiment, the waxes of plant origin with a melting point above 25°C according to the invention are present in a content ranging from 0.01% to 20% by weight, in particular from 0.05% to 10% by weight, more particularly from 0.1% to 5% by weight, relative to the total weight of the composition.

According to a preferred embodiment, a composition according to the invention comprises from 0.01% to 10% by weight of hydrogenated jojoba oil, in particular from 0.05% to 8% by weight, more particularly from 0.1% to 5% by weight, and preferably from 0.5% to 3% by weight, relative to the total weight of the composition.

### Oily phase

The composition according to the invention comprises an oily phase. The proportion of the oily phase of the emulsion may range, for example, from 0.1% to 50% by weight, preferably from 1% to 30% by weight, even more preferentially from 2% to 20% by weight and better still from 4% to 15% by weight.

This indicated amount does not comprise the content of anionic surfactants as defined previously.

For the purposes of the invention, the oily phase includes any fatty substance that is liquid at room temperature and atmospheric pressure, generally oils, or that is solid at room temperature and atmospheric pressure, like waxes, or any pasty compound, which are present in said composition, with the exception of the fatty alcohols as defined previously.

### Aqueous phase

The composition according to the invention comprises an aqueous phase.

The aqueous phase of the composition in accordance with the invention comprises at least water and the polyol(s) as defined previously. The amount of aqueous phase may range from 50% to 99% by weight, preferably from 60% to 97% by weight and better still from 70% to 95% by weight, relative to the total weight of the composition. According to one embodiment, the amount of aqueous phase may range from 50% to 92% by weight, preferably from 60% to 90% by weight and better still from 70% to 90% by weight, relative to the total weight of the composition. The amount of water may represent all or some of the aqueous phase and it is generally at least 25% by weight relative to the total weight of the composition, preferably at least 35% by weight, better still at least 40% by weight.

The aqueous (or hydrophilic) phase of the composition according to the invention may also contain any water-soluble or water-dispersible additive.

Water-soluble additives that may also be mentioned include primary alcohols, i.e. an alcohol including from 1 to 6 carbon atoms, such as ethanol and isopropanol. It is preferably ethanol. The addition of such an alcohol may notably be suitable when the composition according to the invention is used as a product for the body or the hair.

The amount of water-soluble or water-dispersible additives in the composition of the invention may range, for example, from 0% to 50% by weight, preferably from 0.5% to 30% by weight and even more preferentially from 1% to 20% by weight, relative to the total weight of the composition.

According to a particular embodiment of the invention, the pH of the composition is between 4 and 9 and more preferentially between 4.5 and 7.

### Cosmetic active agents and adjuvants

The composition according to the invention may also comprise at least one additional cosmetic active agent.

In the context of the present invention, the term "additional active agent" means a compound which has, by itself, that is to say not requiring the intervention of an external agent in order to activate it, a biological activity.

The additional active agent(s) may notably be chosen from:
- vitamins and derivatives thereof, notably esters thereof, such as tocopherol (vitamin E) and esters thereof (for instance tocopheryl acetate), ascorbic acid (vitamin C) and derivatives thereof, panthenol, niacinamide or vitamin B3;
- known anti-ageing active agents, such as hyaluronic acid compounds, and notably sodium hyaluronate, retinol and derivatives thereof, salicylic acid compounds and in particular 5-n-octanoylsalicylic acid (capryloylsalicylic acid), caffeine, adenosine, C-β-D-xylopyranoside-2-hydroxypropane and the sodium salt of (3-hydroxy-2-pentylcyclopentyl)acetic acid;
- sunscreens;
- menthol;
- and mixtures thereof.

The composition according to the invention may also include at least one additive chosen from the usual adjuvants in the cosmetic field, such as preserving agents, fragrances, film-forming polymers, thickening polymers, pH adjusters (acids or bases), preserving agents, fillers, dyes, nacres, pigments and dispersants, and mixtures thereof.

The additional active agent used in the composition according to the invention may represent from 0.0001% to 40%, preferably from 0.01% to 3%, better still from 0.01% to 20% by weight, or from 0.01% to 15% by weight relative to the total weight of the composition. Needless to say, a person skilled in the art will take care to select this or these optional additional compound(s), and/or the amount thereof, such that the advantageous properties of the composition according to the invention are not, or are not substantially, adversely affected by the envisioned addition.

### Cosmetic composition

The composition used according to the invention may be in any galenic form normally used in the cosmetics field.

The composition according to the invention is in the form of an oil-in-water emulsion. It includes an oily phase dispersed in an aqueous phase.

According to a preferred embodiment, the compositions in accordance with the invention are not solid.

According to another preferred embodiment, the compositions in accordance with the invention are not in stick form.

According to a particularly preferred embodiment, the compositions according to the invention are not solid and are not in stick form.

Advantageously, the compositions according to the invention are in the form of a non-solid galenic, ranging from a serum texture to a creamy texture that can be kept in a jar.

Advantageously, the viscosity of a composition of the invention ranges between 100 cP (centipoises), that is to say 0.1 Pa.s (Pascal.second) (mobile internal measurement 2, 21UD - fluid milk type) and 20 040 cP, that is to say 20.04 Pa.s (mobile internal measurement 4, 85UD - heavy cream type) measured at 25°C.

The viscosity of a composition of the invention can be measured by any method known to the skilled person, and in particular by the following conventional method. For example, measurement can be carried out at 25°C using a Contraves TV or Rheomat RM180, equipped with a mobile rotating at 200 rpm. The person skilled in the art can choose the mobile used to measure viscosity, for example from M1, M2 or M4 mobiles, on the basis of his general knowledge, so as to be able to carry out the measurement.

In addition, the composition according to the invention may be more or less fluid and may have the appearance of a gel, a white or coloured cream, an ointment, a milk, a lotion, a serum, a paste or a mousse.

More particularly, the composition according to the invention is intended for topical application.

The composition according to the invention may comprise any constituent usually employed in the envisaged topical application and administration.

Preferably, such a composition is not intended to be rinsed off after application.

The composition according to the invention may be intended to be applied to the skin. Preferably, the skin is the skin of the face and/or of the body, in particular of the face and/or of the neck and/or of the hands, preferably of the face and/or of the neck.

As specified, the composition according to the invention is advantageously in the form of an oil-in-water emulsion.

The composition may alternatively be in the form of a face and/or body care or makeup product, and may be packaged, for example, in the form of a cream in a jar or a fluid in a tube or a pump bottle or a dropper bottle.

The composition according to the invention may be manufactured via any known process generally used in the cosmetics field.

The ingredients are mixed before forming, in the order and under conditions readily determined by a person skilled in the art.

### Processes

According to one of its aspects, the invention relates to a process for the cosmetic treatment of a keratin material in which a composition as defined in the present invention is applied to the keratin material.

The cosmetic processes considered according to the invention are non-therapeutic. The cosmetic process of the invention is performed by topically administering a composition according to the invention.

Topical administration consists of the external application to the skin of cosmetic compositions according to the usual techniques for the use of these compositions.

By way of illustration, the cosmetic process according to the invention may be performed by topical application, for example daily, of at least one composition according to the invention, which may be formulated, for example, in the form of a gel, a white or coloured cream, an ointment, a milk, a lotion, a serum, a paste or a foam.

According to one embodiment, the application is repeated, for example, once or twice a day for one or more days and generally over an extended period of at least 4 weeks, or even 4 to 15 weeks, with, where appropriate, one or more periods of stoppage.

According to one embodiment, the application is daily (once a day) and generally over an extended period of at least 4 weeks, or even 4 to 15 weeks, with, where appropriate, one or more periods of stoppage.

According to an embodiment variant, the composition according to the invention will be applied to areas of skin that have been previously cleansed using a washing solution. Moreover, treatment combinations optionally with topical forms may be envisaged, so as to complement or reinforce the activity of the composition according to the invention.

A topical treatment with the composition according to the invention, combined with an ancillary composition intended for topical application, this ancillary composition possibly being impregnated onto a support such as a wipe, is conceivable.

Thus, the present invention relates in particular to a cosmetic process for treating a keratin material, comprising:
a) a step of topical application to the skin of a washing solution;
b) a step of topical application to the skin of a composition according to the invention; and
c) optionally, a step of topical application of a composition different from the composition applied in step b);
it being possible for steps b) and c) to be performed simultaneously or successively.

Such a composition applied in step c) can be termed a "supplementary composition".

The washing solution may notably be in various forms, such as a solution, an aqueous solution, a lotion, a milky lotion, a cream, a gel, a liquid gel, a paste, a serum, a suspension, a dispersion, a fluid, a milk, an emulsion (O/W or W/O form), or others, preferably in the form of a solution or an aqueous gel.

Throughout the description, including the claims, the terms "between ... and ...", and "ranging from ... to ..." should be understood as meaning limits included, unless otherwise specified.

The examples that follow illustrate the present invention without limiting the scope thereof.

### Examples

### MATERIALS AND METHODS

### Stability measurement

The stability of the compositions may be evaluated according to the following protocol. The properties of the composition to be tested are evaluated immediately after its preparation and 24 hours later.

The composition to be tested is then stored for 2 months at various temperatures: at room temperature or "RT" (about 25°C), at 4°C and at 45°C. The temperature may be regulated using an oven, for example the Bio Concept machine from Firlabo.

The properties are evaluated after 1 month and 2 months of storage under the conditions described above.

The properties evaluated are:
- the pH of the composition, measured using a pH-meter;
- the viscosity of the composition, measured with a viscometer, for example a Rheomat RM 100 viscometer from Lamy Rheology according to the CID-012-02 method;
- the appearance of the composition, in particular its microscopic appearance evaluated by observation of the composition under a light microscope between slide and coverslip, at X10 or even X100 magnification;
- the colour and odour of the composition; and

Phenomena associated with destabilization of the composition will be observed, such as the appearance of creaming, coalescence, sedimentation, flocculation, the formation of a film on the surface, mottling, etc.

The closer the properties of the composition after 1 month and/or 2 months of storage remain to those initially measured at 24h at 25°C, the more the composition can be considered as remaining stable.

In particular, if the composition remains smooth and homogeneous, with no signs of destabilization and with no change in colour or odour, then it is considered to be stable.

### EXAMPLE 1

A composition according to the invention (**I1**) and a composition outside the invention (**C1**), in particular comprising a non-silicone oil content of less than 8% by weight (in this case 5% by weight) relative to the total weight of the composition, were prepared.

These compositions, in the form of oil-in-water emulsions, comprise the compounds detailed in Table 1 below, in contents expressed as weight percentages relative to the total weight of the composition.

**[Table 1]**

| **Phase** | **Compound** | **11 (invention)** | **C1 (outside the invention)** |
|---|---|---|---|
| A | **Cetearyl alcohol** | 6.30 | 6.30 |
| | Preserving agents | 0.50 | 0.50 |
| A | Water | qs 100 | qs 100 |
| A | Chelating agent | 0.25 | 0.25 |
| A | Caprylyl glycol | 0.30 | 0.30 |
| A | **Sodium methyl stearoyl taurate (Nikkol SMT sold by the company Nikko)** | 0.70 | 0.70 |
| B | **Oleyl erucate (Cetiol J 600 sold by the company BASF)** | 7.50 | 2.50 |
| | Nacre | 0.14 | 0.14 |
| | **C15-19 Alkane (Emogreen L15 sold by the company SEPPIC)** | 7.50 | 2.50 |
| C | Glycerol | 20.00 | 20.00 |
| D | Ammonium acryloyldimethyltaurate/VP copolymer (Aristoflex AVC sold by the company Clariant) | 0.35 | 0.35 |
| E | Water | 0.98 | 0.98 |
| E | Citric acid | 0.02 | 0.02 |
| F | Nacre | 0.35 | 0.35 |

### Preparation protocol

In a tank, the components of phase A are heated to 75°C until a homogeneous medium is obtained by paddle stirring without turbomixing. Phase A is turbomixed at 75°C for 10 minutes (3500 rpm). The components of phase B, heated to 75°C with magnetic stirring, are added to this heated mixture, while turbomixing for 10 minutes (3500 rpm). Glycerol is added to this mixture and the resulting mixture is cooled to 40°C with stirring (3500 rpm) and under vacuum. The components of phase D are then added with stirring and under vacuum, followed by the components of the other phases.

Finally, a final homogenization phase is performed down to a temperature of 30°C, with continued stirring and under vacuum, the turbomixing being reduced if necessary.

### EXAMPLE 2

### Results

The stability and the sensory effect of compositions **I1** and **C1** were evaluated. The stability results are reported in Table 2 below.

**[Table 2]**

| **Stability** | **I1 (invention)** | **C1 (outside the invention)** |
|---|---|---|
| **STABILITY 24 hours** | Appearance/Colour/Odour: Supple pink cream | Appearance/Colour/Odour: Supple pink cream |
| | Viscosity | Viscosity |
| | Viscosity (Spindle 3) = T30 sec = 3.16 Pa.s; T10 min = 2.82 Pa.s | Viscosity (Spindle 3) = T30 sec = 3.04 Pa.s; T10 min = 2.99 Pa.s |
| | pH | pH |
| | pH = 6.3 | pH = 6.4 |
| | Light microscopy: | Light microscopy: |
| | Microscopy (X10): | Microscopy (X10): Fine and tight emulsion, sharp edges |
| | LNP Fine and tight emulsion, sharp edges | |
| | | Anisotropy in polarized light |
| | Anisotropy in polarized light | |
| **STABILITY 1M 4°C/RT (25°C)/45°C** | COMPLIANT | COMPLIANT |
| | Appearance/Colour/Odour: compliant | Appearance/Colour/Odour: compliant |
| | Light microscopy: compliant | Light microscopy: compliant |
| **STABILITY 2M 4°C/RT (25°C)/45°C** | COMPLIANT | NON-COMPLIANT |
| | Appearance/Colour/Odour: compliant | Appearance/Colour/Odour: compliant |
| | Light microscopy: at 4°C and at RT: compliant | Light microscopy: at 4°C and at RT: compliant |
| | Viscosity: Compliant | Viscosity: non-compliant |
| | at RT (25°C): | at RT (25°C): |
| | Viscosity (spindle 3): T 30 sec = 2.53 Pa.s; T 10 min = 2.66 Pa.s | Viscosity (spindle 3): T 30 sec = 2.20 Pa.s; T 10 min = 2.74 Pa.s |
| | at 45°C (returned to RT 25°C before measuring the viscosity): | at 45°C (returned to RT 25°C before measuring the viscosity): |
| | Viscosity (spindle 3) = T 30 sec = 2.95 Pa.s; T 10 min = 3.04 Pa.s | Viscosity (spindle 3) = T 30 sec = 0.90 Pa.s; T 10 min = 2.57 Pa.s |
| | pH | pH |
| | pH at RT (25°C) = 6.2 | pH at RT (25°C) = 6.5 |
| | pH at 45°C (returned to RT 25°C before measuring the pH) = 6.3 | pH at 45°C (returned to RT 25°C before measuring the pH) = 6.5 |

In conclusion, only the composition according to the invention **I1** is stable after 2 months, whether at 4°C, at room temperature or at 45°C. Specifically, it is compliant in terms of appearance, colour, odour, viscosity and pH.

Composition C1, which is outside the invention, is non-compliant after 2 months in terms of viscosity.

As regards the sensory evaluation, a panel of nine experts took part in this study on the basis of a tactile and visual evaluation. The compositions were applied to the back of the hand. Composition **I1** according to the invention was found to afford good glidance. Moreover, it is described as being sparingly tacky and has little soapiness.

### EXAMPLE 3

### Preparation of a composition outside the invention

A composition outside the invention **(C2)** was prepared. This composition, in the form of an oil-in-water emulsion, comprises the compounds detailed in Table 3 below, in contents expressed as weight percentages relative to the total weight of the composition. The composition has a polyol content of less than 15% by weight relative to the total weight of the composition.

**[Table 3]**

| **Phase** | **Compound** | **C2 (outside the invention)** |
|---|---|---|
| A | **Cetearyl alcohol** | 6.30 |
| | Preserving agents | 0.50 |
| A | Water | qs 100 |
| A | Chelating agent | 0.25 |
| A | Caprylyl glycol | 0.30 |
| A | **Sodium methyl stearoyl taurate (Nikkol SMT sold by the company Nikko)** | 0.70 |
| B | **Oleyl erucate (Cetiol J 600 sold by the company BASF)** | 7.50 |
| | Nacre | 0.14 |
| | **C15-19 Alkane (Emogreen L15 sold by the company SEPPIC)** | 7.50 |
| C | Glycerol | 12.00 |
| D | Ammonium acryloyldimethyltaurate/VP copolymer (Aristoflex AVC sold by the company Clariant) | 0.35 |
| E | Water | 0.98 |
| E | Citric acid | 0.02 |
| F | Nacre | 0.35 |

### Preparation protocol

The same protocol as that used in Example 1 is used in this example.

### EXAMPLE 4

### Results

The stability of compositions **I1** and **C2** was evaluated. The stability results are reported in Table 4 below.

**[Table 4]**

| **Stability** | **11 (invention)** | **C2 (outside the invention)** |
|---|---|---|
| **STABILITY 24 hours** | Appearance/Colour/Odour: Supple pink cream | Appearance/Colour/Odour: Supple pink cream |
| | Viscosity | Viscosity |
| | Viscosity (Spindle 3) = T30 sec = 3.16 Pa.s; T10 min = 2.82 Pa.s | Viscosity (Spindle 3) = T30 sec = 3.07 Pa.s; T10 min = 2.70 Pa.s |
| | pH | pH |
| | pH = 6.3 | pH = 6.4 |
| | Light microscopy: | Light microscopy: |
| | Microscopy (X10): LNP Fine and tight emulsion, sharp edges | Microscopy (X10): Fine and tight emulsion, sharp edges |
| | | Anisotropy in polarized light |
| | Anisotropy in polarized light | |
| **STABILITY 1M 4°C/RT (25°C)/45°C** | COMPLIANT | COMPLIANT |
| | Appearance/Colour/Odour: compliant | Appearance/Colour/Odour: compliant |
| | Light microscopy: compliant | Light microscopy: compliant |
| **STABILITY 2M 4°C/RT (25°C)/45°C** | COMPLIANT | NON-COMPLIANT |
| | Appearance/Colour/Odour: compliant | Appearance/Colour/Odour: compliant |
| | Light microscopy: compliant | Light microscopy: compliant |
| | Viscosity: Compliant | Viscosity: non-compliant |
| | at RT (25°C): | at RT (25°C): |
| | Viscosity (spindle 3): T 30 sec = 2.53 Pa.s; T 10 min = 2.66 Pa.s | Viscosity (spindle 3): T 30 sec = 0.07 Pa.s; T 10 min = 2.49 Pa.s |
| | at 45°C (returned to RT 25°C before measuring the viscosity): | at 45°C (returned to RT 25°C before measuring the viscosity): |
| | Viscosity (spindle 3) = T 30 sec = 2.95 Pa.s; T 10 min = 3.04 Pa.s | Viscosity (spindle 3) = T 30 sec = 2.07 Pa.s; T 10 min = 2.07 Pa.s |
| | pH | pH |
| | pH at RT (25°C) = 6.2 | pH at RT (25°C) = 6.6 |
| | pH at 45°C (returned to RT 25°C before measuring the pH) = 6.3 | pH at 45°C (returned to RT 25°C before measuring the pH) = 6.6 |

In conclusion, only the composition according to the invention **I1** is stable after 2 months, whether at 4°C, at room temperature or at 45°C. Specifically, it is compliant in terms of appearance, colour, odour, viscosity and pH.

Composition C2, which is outside the invention, is non-compliant after 2 months in terms of viscosity.

### EXAMPLE 5

### Preparation of the compositions according to the invention

Compositions according to the invention (**I2**), (**I3**), (**I4**) and (**I5**) were prepared. These compositions, in the form of oil-in-water emulsions, comprise the compounds detailed in Table 5 below, in contents expressed as weight percentages relative to the total weight of the composition.

**[Table 5]**

| **Phase** | **Compound** | **I2** | **I3** | **I4** | **I5** |
|---|---|---|---|---|---|
| A | Water | qs | qs | qs | qs |
| | | 100 | 100 | 100 | 100 |
| A | **Cetearyl alcohol** | **4.50** | **4.50** | **4.50** | **4.50** |
| | Preserving agent | 0.50 | 0.50 | 0.50 | 0.50 |
| A | **Caprylyl glycol** | **0.30** | **0.30** | **0.30** | **0.30** |
| | Poly C10-30 alkyl acrylate | - | 0.57 | - | 0.38 |
| A | Chelating agent | 0.25 | 0.25 | 0.25 | 0.25 |
| A | **Sodium methyl stearoyl taurate (Nikkol SMT sold by the company Nikko)** | **0.50** | **0.50** | **0.50** | **0.50** |
| B | Octyldodecanol | - | 1.50 | 1.50 | - |
| B | **Oleyl erucate (Cetiol J 600 sold by the company BASF)** | **5.00** | **5.00** | **5.00** | **5.00** |
| | Capryloyl salicylic acid | - | 0.15 | 0.15 | - |
| B | Hydrogenated jojoba oil | 1.00 | 1.00 | 1.00 | 1.00 |
| B | **C15-19 alkane (Emogreen L15 sold by the company SEPPIC)** | **5.00** | **5.00** | **5.00** | **5.00** |
| C | **Glycerol** | **20.00** | **20.00** | **20.00** | **20.00** |
| D | Xanthan gum | - | 0.10 | 0.10 | 0.10 |
| | Sodium hyaluronate | | 0.10 | 0.10 | 0.10 |
| D | Ammonium acryloyldimethyltaurate/VP copolymer (Aristoflex AVC sold by the company Clariant) | 0.35 | - | 0.50 | - |
| | Ammonium acryloyldimethyltaurate/Steareth-25 methacrylate crosspolymer (Aristoflex HMS sold by the company Clariant) | - | 0.50 | - | 0.50 |
| E | Hydroxypropyl tetrahydropyrantriol | - | 4.30 | 4.30 | 4.30 |
| E | Sodium dilauramidoglutamide lysine | - | 0.35 | - | 0.35 |
| E | Citric acid | 0.02 | - | - | 0.02 |
| F | Niacinamide | - | 3.00 | 3.00 | - |
| F | Nacre | - | - | 2.55 | - |
| G | Silica silylate (Dowsil VM-2270 Aerogel Fine Particles sold by the company Dow Corning) | - | 0.14 | - | 0.09 |
| G | Cellulose (Cellulobeads USF sold by the company Daito Kasei Kogyo) | - | 0.70 | - | 0.47 |
| G | Nacres | - | 2 | - | - |
| H | Denatured alcohol | - | 3.00 | - | 3.00 |
| H | Menthol | - | 0.15 | - | 0.15 |

### Preparation protocol

The same protocol as that used in Example 1 is used in this example.

Compositions **(12)** to **(I5)** have a good level of glidance and are sparingly tacky, while at the same time having little soapiness.

## Claims

1. Composition, preferably a cosmetic composition, in the form of an oil-in-water emulsion, in which an oily phase is dispersed in an aqueous phase, comprising:
(a) at least one saturated C16-C22 and preferably C16-C18 fatty alcohol;
(b) at least one anionic surfactant including at least one C16-C22 and preferably C16-C18 hydrocarbon-based chain;
(c) at least one polyol present in an amount strictly greater than 15% by weight relative to the total weight of the composition, and
(d) at least one non-silicone oil present in an amount of greater than or equal to 8% by weight relative to the total weight of the composition,
the composition being free of silicone compounds.

2. Composition according to Claim 1, in which the anionic surfactant(s) contain only one C16-C22 hydrocarbon-based chain.

3. Composition according to either of Claims 1 and 2, in which the saturated fatty alcohol(s) are chosen from cetyl alcohol or hexadecanol (C₁₆), stearyl alcohol or octadecanol (C₁₈), or behenyl alcohol (C₂₂) which are solid at room temperature and advantageously bear a chain-end -OH group.

4. Composition according to any one of Claims 1 to 3, in which the saturated fatty alcohol(s) are chosen from cetyl alcohol, stearyl alcohol and mixtures thereof such as cetylstearyl alcohol.

5. Composition according to any one of Claims 1 to 4, comprising a mixture of C₁₆ and C₁₈ fatty alcohols, preferably in a C₁₆/C₁₈ mass ratio ranging from 20/80 to 80/20, and advantageously in a C₁₆/C₁₈ mass ratio equal to 50/50.

6. Composition according to any one of Claims 1 to 5, in which the amount of fatty alcohol is between 2.5% and 10% by weight, preferably between 2.5% and 8% by weight and even more preferentially between 2.7% and 7% by weight relative to the total weight of the composition.

7. Composition according to any one of Claims 1 to 6, in which the anionic surfactant(s) including at least one C16-C22 hydrocarbon-based chain are chosen from surfactants comprising at least one sulfonate function, acylglutamates, and mixtures thereof, or are chosen from the surfactants of general formula (I): RCOY(CH₂)ₙSO₃M in which R represents a saturated, linear or branched C16-C22 alkyl group; Y represents -O- or -NR₁- with R₁ representing a linear or branched C1-C3 alkyl group; M is chosen from the group formed by hydrogen, alkali metals, alkaline-earth metals, the ammonium group and organic amines; n is an integer ranging from 1 to 3 or is sodium N-stearoyl-N-methyltaurate.

8. Composition according to any one of Claims 1 to 7, in which the surfactant(s) including at least one C16-C22 hydrocarbon-based chain are present in an amount of between 0.05% and 5% by weight, preferably between 0.1% and 2% by weight and even more preferentially from 0.1% to 1.5% by weight relative to the total weight of the composition.

9. Composition according to any one of Claims 1 to 8, in which the fatty alcohols (a)/anionic surfactants (b) mass ratio is between 10/1 and 6/4, preferably between 19/2 and 7/3 and even more preferentially between 9/1 and 4/1.

10. Composition according to any one of Claims 1 to 9, in which the polyol(s) comprise a number of carbon atoms ranging from 2 to 20 and preferably from 2 to 10.

11. Composition according to the preceding claim, in which the polyol(s) comprise from 2 to 12 hydroxyl functions, preferably from 2 to 8 hydroxyl functions, in particular are chosen from ethylene glycol, propylene glycol, propane-1,3-diol, isoprene glycol, butylene glycol, dipropylene glycol, polypropylene glycol, caprylyl glycol, glycerol, diglycerol, erythritol, pentaerythritol, arabitol, adonitol, sorbitol, dulcitol, maltitol, panthenol and mixtures thereof, preferably glycerol, caprylyl glycol, propylene glycol, dipropylene glycol, butylene glycol, propane-1,3-diol and mixtures thereof, and even more preferentially glycerol, caprylyl glycol and mixtures thereof.

12. Composition according to Claim 10 or 11, in which the polyol(s) are present in an amount of between 15.1% and 50% by weight, preferably between 18% and 45% by weight, even more preferentially between 19% and 40% by weight, better still between 20% and 35% by weight, relative to the total weight of the composition.

13. Composition according to any one of Claims 1 to 124, in which the non-silicone oil(s) are chosen from branched or linear alkanes comprising more than 8 carbon atoms, hydrocarbon-based oils of animal origin, linear or branched hydrocarbons of mineral or synthetic origin, hydrocarbon-based oils of plant origin, synthetic ethers containing from 10 to 40 carbon atoms, and synthetic esters of formula R₁COOR₂, in which R₁ represents a residue of a linear or branched fatty acid including from 1 to 40 carbon atoms and R₂ represents a hydrocarbon-based chain, notably a branched chain containing from 1 to 40 carbon atoms, on condition that R₁ + R₂ is greater than or equal to 10, polyol esters and pentaerythritol esters, diol dimer and diacid dimer esters, fatty amides, fatty alcohols that are liquid at room temperature, with a branched and optionally unsaturated carbon-based chain containing from 12 to 26 carbon atoms, higher C₁₉-C₂₂ fatty acids, carbonates and mixtures thereof; in particular from oleyl erucate, branched or linear alkanes comprising more than 8 carbon atoms, and mixtures thereof; more particularly from oleyl erucate, a mixture of alkanes of 15 to 19 carbon atoms, and mixtures thereof.

14. Composition according to any one of Claims 1 to 13, in which the non-silicone oil(s) are present in an amount of between 8% and 40% by weight, preferably between 9% and 25% by weight and even more preferentially between 10% and 15% by weight relative to the total weight of the composition.

15. Cosmetic process for treating a keratin material, in which a composition as defined in any one of Claims 1 to 14 is applied to the keratin material.

## Patentansprüche

1. Zusammensetzung, vorzugsweise kosmetische Zusammensetzung, in Form einer Öl-in-Wasser-Emulsion, wobei eine ölige Phase in einer wässrigen Phase dispergiert ist, umfassend:
(a) mindestens einen gesättigten C16-C22- und vorzugsweise C16-C18-Fettalkohol;
(b) mindestens ein anionisches Tensid mit mindestens einer kohlenwasserstoffbasierten C16-C22- und vorzugsweise C16-C18-Kette;
(c) mindestens ein Polyol, das in einer Menge von streng mehr als 15 Gew.-%, bezogen auf das Gesamtgewicht der Zusammensetzung, vorhanden ist, und
(d) mindestens ein Nichtsilikon-Öl, das in einer Menge größer als oder gleich 8 Gew.-%, bezogen auf das Gesamtgewicht der Zusammensetzung, vorhanden ist,
wobei die Zusammensetzung frei von Silikonverbindungen ist.

2. Zusammensetzung nach Anspruch 1, wobei das anionische Tensid bzw. die anionischen Tenside nur eine kohlenwasserstoffbasierte C16-C22-Kette enthält bzw. enthalten.

3. Zusammensetzung nach einem der Ansprüche 1 bis 2, wobei der gesättigte Fettalkohol bzw. die gesättigten Fettalkohole aus Cetylalkohol oder Hexadecanol (C₁₆), Stearylalkohol oder Octadecanol (C₁₈) oder Behenylalkohol (C₂₂), die bei Raumtemperatur fest sind und vorteilhafterweise eine -OH-Gruppe am Kettenende tragen, ausgewählt ist bzw. sind.

4. Zusammensetzung nach einem der Ansprüche 1 bis 3, wobei der gesättigte Fettalkohol bzw. die gesättigten Fettalkohole aus Cetylalkohol, Stearylalkohol und Mischungen davon wie Cetylstearylalkohol ausgewählt ist bzw. sind.

5. Zusammensetzung nach einem der Ansprüche 1 bis 4, umfassend eine Mischung von C₁₆- und C₁₈-Fettalkoholen, vorzugsweise in einem C₁₆/C₁₈-Massenverhältnis im Bereich von 20/80 bis 80/20 und vorteilhafterweise in einem C₁₆/C₁₈-Massenverhältnis gleich 50/50.

6. Zusammensetzung nach einem der Ansprüche 1 bis 5, wobei die Menge an Fettalkohol zwischen 2,5 Gew.-% und 10 Gew.-%, vorzugsweise zwischen 2,5 Gew.-% und 8 Gew.-% und noch weiter bevorzugt zwischen 2,7 Gew.-% und 7 Gew.-%, bezogen auf das Gesamtgewicht der Zusammensetzung, liegt.

7. Zusammensetzung nach einem der Ansprüche 1 bis 6, wobei das anionische Tensid bzw. die anionischen Tenside mit mindestens einer kohlenwasserstoffbasierten C16-C22-Kette aus Tensiden, die mindestens eine Sulfonatfunktion umfassen, Acylglutamaten und Mischungen davon ausgewählt sind, oder ausgewählt ist bzw. sind aus den Tensiden der allgemeinen Formel (I): RCOY(CH₂)ₙSO₃M, wobei R für eine gesättigte, lineare oder verzweigte C16-C22-Alkylgruppe steht; Y für -O- oder -NR₁- steht, wobei R₁ für eine lineare oder verzweigte C1-C3-Alkylgruppe steht; M aus der Gruppe ausgewählt ist, die durch Wasserstoff, Alkalimetalle, Erdalkalimetalle, die Ammoniumgruppe und organische Amine gebildet wird; n eine Ganzzahl im Bereich von 1 bis 3 ist; oder Natrium-N-stearoyl-N-methyltaurat ist.

8. Zusammensetzung nach einem der Ansprüche 1 bis 7, wobei das Tensid bzw. die Tenside mit mindestens einer kohlenwasserstoffbasierten C16-C22-Kette in einer Menge zwischen 0,05 Gew.-% und 5 Gew.-%, vorzugsweise zwischen 0,1 Gew.-% und 2 Gew.-% und noch weiter bevorzugt zwischen 0,1 Gew.-% und 1,5 Gew.-%, bezogen auf das Gesamtgewicht der Zusammensetzung, vorliegt bzw. vorliegen.

9. Zusammensetzung nach einem der Ansprüche 1 bis 8, wobei das Massenverhältnis von Fettalkoholen (a) zu anionischen Tensiden (b) zwischen 10/1 und 6/4, bevorzugt zwischen 19/2 und 7/3 und noch weiter bevorzugt zwischen 9/1 und 4/1 liegt.

10. Zusammensetzung nach einem der Ansprüche 1 bis 9, wobei das Polyol bzw. die Polyole eine Zahl an Kohlenstoffatomen im Bereich von 2 bis 20 und vorzugsweise von 2 bis 10 umfasst bzw. umfassen.

11. Zusammensetzung nach dem vorhergehenden Anspruch, wobei das oder die Polyol(e) 2 bis 12 Hydroxylfunktionen, vorzugsweise 2 bis 8 Hydroxylfunktionen umfassen, insbesondere ausgewählt sind aus Ethylenglykol, Propylenglykol, Propan-1,3-diol, Isoprenglykol, Butylenglykol, Dipropylenglykol, Polypropylenglykol, Caprylylglykol, Glycerin, Diglycerin, Erythrit, Pentaerythrit, Arabitol, Adonitol, Sorbit, Dulcitol, Maltit, Panthenol und Mischungen davon, vorzugsweise Glycerin, Caprylylglykol, Propylenglykol, Dipropylenglykol, Butylenglykol, Propan-1,3-diol und Mischungen davon, noch bevorzugter Glycerin, Caprylylglykol und Mischungen davon.

12. Zusammensetzung nach einem der Ansprüche 10 bis 11, wobei das bzw. die Polyol(e) in einer Menge zwischen 15,1 und 50 Gew.-%, vorzugsweise zwischen 18 und 45 Gew.-%, noch weiter bevorzugt zwischen 19 und 40 Gew.-% und sogar noch besser zwischen 20 und 35 Gew.-%, bezogen auf das Gesamtgewicht der Zusammensetzung, vorliegt bzw. vorliegen.

13. Zusammensetzung nach einem der Ansprüche 1 bis 12, wobei das oder die Nicht-Silikonöl(e) ausgewählt sind aus verzweigten oder linearen Alkanen mit mehr als 8 Kohlenstoffatomen, Ölen auf Kohlenwasserstoffbasis tierischen Ursprungs, linearen oder verzweigten Kohlenwasserstoffen mineralischen oder synthetischen Ursprungs, kohlenwasserstoffbasierten Ölen pflanzlichen Ursprungs, synthetischen Ethern mit 10 bis 40 Kohlenstoffatomen und synthetischen Estern der Formel R₁COOR₂, wobei R₁ für einen Rest einer linearen oder verzweigten Fettsäure mit 1 bis 40 Kohlenstoffatomen steht und R₂ für eine kohlenwasserstoffbasierte Kette, insbesondere eine verzweigte Kette mit 1 bis 40 Kohlenstoffatomen steht, unter der Bedingung, dass R₁ + R₂ größer oder gleich 10 ist, Polyolestern und Pentaerythritestern, Dioldimer- und Disäuredimerestern, Fettamiden, Fettalkoholen, die bei Raumtemperatur flüssig sind, mit einer verzweigten und gegebenenfalls ungesättigten kohlenwasserstoffbasierten Kette mit 12 bis 26 Kohlenstoffatomen, höheren C₁₉-C₂₂-Fettsäuren, Carbonaten und deren Mischungen, insbesondere aus Oleylerucat, verzweigten oder linearen Alkanen mit mehr als 8 Kohlenstoffatomen und Mischungen davon, insbesondere aus Oleylerucat, einem Gemisch von Alkanen mit 15 bis 19 Kohlenstoffatomen und deren Mischungen.

14. Zusammensetzung nach einem der Ansprüche 1 bis 13, wobei das oder die Nicht-Silikonöl(e) in einer Menge zwischen 8 Gew.-% und 40 Gew.-%, vorzugsweise zwischen 9 Gew.-% und 25 Gew.-% und noch weiter bevorzugt zwischen 10 Gew.-% und 15 Gew.-%, bezogen auf das Gesamtgewicht der Zusammensetzung, vorliegt bzw. vorliegen.

15. Kosmetisches Verfahren zur Behandlung eines Keratinmaterials, bei dem eine Zusammensetzung gemäß einem der Ansprüche 1 bis 14 auf das Keratinmaterial aufgebracht wird.

## Revendications

1. Composition, de préférence composition cosmétique, sous la forme d'une émulsion huile-dans-eau, dans laquelle une phase huileuse est dispersée dans une phase aqueuse, comprenant :
(a) au moins un alcool gras saturé en C16-C22 et de préférence en C16-C18 ;
(b) au moins un tensioactif anionique comprenant au moins une chaîne à base d'hydrocarbure en C16-C22, de préférence en C16-C18 ;
(c) au moins un polyol présent en une quantité strictement supérieure à 15 % en poids par rapport au poids total de la composition, et
(d) au moins une huile non siliconée présente en une quantité supérieure ou égale à 8 % en poids par rapport au poids total de la composition,
la composition étant exempte de composés de silicone.

2. Composition selon la revendication 1, dans laquelle le ou les tensioactifs anioniques contiennent seulement une chaîne à base d'hydrocarbure en C16-C22.

3. Composition selon l'une ou l'autre des revendications 1 et 2, dans laquelle le ou les alcools gras saturés sont choisis parmi l'alcool cétylique ou hexadécanol (C₁₆), l'alcool stéarylique ou octadécanol (C₁₈), ou l'alcool béhénylique (C₂₂) qui sont solides à température ambiante et portent avantageusement un groupe -OH en extrémité de chaîne.

4. Composition selon l'une quelconque des revendications 1 à 3, dans laquelle le ou les alcools gras saturés sont choisis parmi l'alcool cétylique, l'alcool stéarylique et leurs mélanges tels que l'alcool cétylstéarylique.

5. Composition selon l'une quelconque des revendications 1 à 4, comprenant un mélange d'alcools gras en C₁₆ et en C₁₈, de préférence en un rapport massique C₁₆/C₁₈ allant de 20/80 à 80/20, et avantageusement en un rapport massique C₁₆/C₁₈ égal à 50/50.

6. Composition selon l'une quelconque des revendications 1 à 5, dans laquelle la quantité d'alcool gras est comprise entre 2,5 % et 10 % en poids, de préférence entre 2,5 % et 8 % en poids, et encore plus préférentiellement entre 2,7 % et 7 % en poids par rapport au poids total de la composition.

7. Composition selon l'une quelconque des revendications 1 à 6, dans laquelle le ou les tensioactifs anioniques comprenant au moins une chaîne à base d'hydrocarbure en C16-C22 sont choisis parmi des tensioactifs comprenant au moins une fonction sulfonate, des acylglutamates et leurs mélanges, ou sont choisis parmi les tensioactifs de formule générale (I) : RCOY(CH₂)ₙSO₃M dans laquelle R représente un groupe alkyle saturé linéaire ou ramifié en C16-C22 ; Y représente -O- ou -NR₁-, R₁ représentant un groupe alkyle linéaire ou ramifié en C1-C3 ; M est choisi dans le groupe formé par hydrogène, les métaux alcalins, les métaux alcalino-terreux, le groupe ammonium et des amines organiques ; n est un entier allant de 1 à 3, ou est le N-stéaroyl-N-méthyltaurate de sodium.

8. Composition selon l'une quelconque des revendications 1 à 7, dans laquelle le ou les tensioactifs comprenant au moins une chaîne à base d'hydrocarbure en C16-C22 sont présents en quantité comprise entre 0,05 et 5 % en poids, de préférence entre 0,1 et 2 % en poids, et encore plus préférentiellement de 0,1 % à 1,5 % en poids par rapport au poids total de la composition.

9. Composition selon l'une quelconque des revendications 1 à 8, dans laquelle le rapport massique alcools gras (a)/tensioactifs anioniques (b) est compris entre 10/1 et 6/4, de préférence entre 19/2 et 7/3 et encore plus préférentiellement entre 9/1 et 4/1.

10. Composition selon l'une quelconque des revendications 1 à 9, dans laquelle le ou les polyols comprennent un nombre d'atomes de carbone allant de 2 à 20, de préférence de 2 à 10.

11. Composition selon la revendication précédente dans laquelle le ou les polyols comprennent de 2 à 12 fonctions hydroxyle, de préférence de 2 à 8 fonctions hydroxyle, en particulier sont choisis parmi l'éthylène glycol, le propylène glycol, le propane-1,3-diol, l'isoprène glycol, le butylène glycol, le dipropylène glycol, le polypropylène glycol, le caprylyl glycol, le glycérol, le diglycérol, l'érythritol, le pentaérythritol, l'arabitol, l'adonitol, le sorbitol, le dulcitol, le maltitol, le panthénol et leurs mélanges, de préférence le glycérol, le caprylyl glycol, le propylène glycol, le dipropylène glycol, le butylène glycol, le propane-1,3-diol et leurs mélanges, et plus particulièrement le glycérol, le caprylyl glycol et leurs mélanges.

12. Composition selon la revendication 10 ou 11, dans laquelle le ou les polyols sont présents en une quantité comprise entre 15,1 % et 50 % en poids, de préférence entre 18 % et 45 % en poids, encore plus préférentiellement entre 19 % et 40 % en poids, mieux encore entre 20 % et 35 % en poids par rapport au poids total de la composition.

13. Composition selon l'une quelconque des revendications 1 à 12, dans laquelle la ou les huiles non siliconée sont choisies parmi les alcanes ramifiés ou linéaires comprenant plus de 8 atomes de carbone, les huiles à base d'hydrocarbure d'origine animale, les hydrocarbures linéaires ou ramifiés d'origine minérale ou synthétique, les huiles hydrocarbonées d'origine végétale, les éthers synthétiques contenant de 10 à 40 atomes de carbone et les esters synthétiques de formule R₁COOR₂, dans lesquels R₁ représente un radical d'acide gras linéaire ou ramifié comprenant de 1 à 40 atomes de carbone et R₂ représente une chaîne hydrocarbonée, notamment une chaîne ramifiée contenant de 1 à 40 atomes de carbone, à condition que R₁ + R₂ soit supérieur ou égal à 10, les esters de polyol et les esters de pentaérythritol, les esters de dimères de diol et de dimères de diacide, les amides gras, les alcools gras liquides à température ambiante, comportant une chaîne carbonée ramifiée et éventuellement insaturée contenant de 12 à 26 atomes de carbone, les acides gras supérieurs en C₁₉-C₂₂, les carbonates et leurs mélanges ; en particulier parmi l'érucate d'oléyle, des alcanes ramifiés ou linéaires comprenant plus de 8 atomes de carbone et leurs mélanges ; plus particulièrement parmi l'érucate d'oléyle, un mélange d'alcanes de 15 à 19 atomes de carbone, et leurs mélanges.

14. Composition selon l'une quelconque des revendications 1 à 13, dans laquelle l'huile ou les huiles non de silicone sont présentes en une quantité comprise entre 8 et 40 % en poids, de préférence entre 9 et 25 % en poids, et encore plus préférentiellement entre 10 % et 15 % en poids par rapport au poids total de la composition.

15. Procédé de traitement cosmétique d'une matière kératinique, dans lequel une composition telle que définie dans l'une quelconque des revendications 1 à 14 est appliquée sur la matière kératinique.
